# EUROPEAN PATENT SPECIFICATION

(11) **EP 2 654 759 B1**
(45) Date of publication and mention of the grant of the patent: **18.10.2017**
(21) Application number: 11852056.8
(22) Date of filing: 14.11.2011
(51) Int. Cl.: A61Q 1/12, A61K 8/86, A61K 8/89, A61K 8/02, A61K 8/90

(54) **LONG WEARING POWDER-BASED COSMETIC COMPOSITIONS**
LANGE HALTENDE KOSMETISCHE ZUSAMMENSETZUNGEN AUF PULVERBASIS
COMPOSITIONS COSMÉTIQUES À BASE DE POUDRE LONGUE TENUE

(30) Priority: 22.12.2010 US 201061426215 P
(43) Date of publication of application: 30.10.2013
(73) Proprietor: Avon Products, Inc., New York, NY 10017 (US)
(72) Inventor: CRUZ, Kathy, Riverdale New York 07457 (US); KYROU, Christos D., Goshen New York 10924 (US); CASTO, Josey, New Milford New Jersey 07646 (US); SHAH, Arvind N., Suffern New York 10901 (US); FAVA, Collette, Yonkers New York 10704 (US)
(74) Representative: Dr. Weitzel & Partner
(86) International application number: PCT/US2011/060516
(87) International publication number: WO 2012/087453

(56) References cited:
- WO-A1-2005/039512
- WO-A1-2006/113882
- WO-A2-2007/078825
- US-A- 6 083 516
- US-A1- 2003 133 895
- US-A1- 2006 008 441
- US-A1- 2007 055 014
- US-A1- 2007 258 934
- US-A1- 2008 305 068
- US-B2- 6 423 306
- TARA ET AL. INTERNATIONAL COSMETIC DICTIONARY AND HANDBOOK vol. 2, 1997, pages 1657 - 1661, XP008169744

## Description

### FIELD OF THE INVENTION

The present invention relates to generally to powder-based cosmetic compositions having improved wear attributes.

### BACKGROUND

Powder-based cosmetics are well known and are a typical product form for eyeshadows and blushes, to name a few. These products are typically in solid form and comprise a major portion of powdered materials, such as fillers and pigments, together with a binder if the product is intended to be pressed into a tin or the like. However, powder-based cosmetics tend to lack substantivity with the skin and therefore are easily transferred to surfaces and must be reapplied frequently to maintain a fresh look.

There is a need in the art for powder-based cosmetics and personal care products which provide longer wear on a human integument that those of the prior art. It is therefore an object of the invention to provide novel powder-based cosmetic and personal care product having improved wear characteristics, such as durability and/or transfer-resistance.

### SUMMARY OF THE INVENTION

In accordance with the foregoing objectives and others detailed herein, the invention overcomes one or more deficiencies associated with the prior art by providing compositions comprising a thermoplastic elastomer, a non-volatile oil, and a film forming polymer comprising a polypropylene glycol (PPG) backbone as defined in claim 1 which achieve excellent wear properties on a human integument.

In one aspect, a long-wearing powder-based cosmetic composition is provided comprising: (a) from about 75% to about 95% by weight of one or more cosmetic powders; (b) from about 0.01% to about 5% by weight of a thermoplastic elastomer comprising a styrene isobutylene styrene (SIBS) block copolymer; (c) from about 1% to about 20% by weight of a non-volatile oil; and (d) from about 0.01% to about 5% by weight of a film-forming polymer comprising a polypropylene glycol (PPG) backbone. The compositions are in the form of a powder and desirably exhibit longer wear on human skin than an otherwise identical composition that does not include components (b)-(d). The compositions may take any number of forms, but are typically pressed or loose powders.

In preferred implementations, the SIBS block copolymer is present in an amount of about 0.1% to about 1.5% by weight of the total composition and the film-forming polymer comprising a polypropylene glycol (PPG) backbone is present in an amount of about 0.1 % to about 1% by weight of the total composition.

The film-forming polymer comprising a polypropylene glycol (PPG) backbone is preferably a polyurethane polymer, for example, one formed by the reaction of a polypropylene glycol diol and a diisocyanate. The polypropylene glycol diol may have the structure: 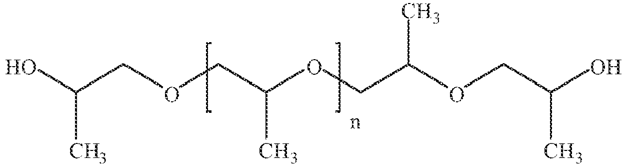 where "n" is an integer from 1 to 100, although "n" will more typically be an integer from 5 to 20. The diisocyante is preferably methylenebis(4-cyclohexyl isocyanate) (SMDI), in which case the film-forming polymer is preferably PEG-12/SMDI Copolymer.

The non-volatile oil may comprises an ester oil, for example Isopropyl Palmitate, C₁₂₋₁₅ alcohols benzoate, and/or a silicone oil, such as a cyclic or linear silicone fluid, although in some embodiments, the compositions are free of cyclic silicones.

In a related aspect, a long-wearing powder-based cosmetic composition is provided comprising: (a) from about 75% to about 95% by weight of one or more cosmetic powders comprising sericite and at least one other cosmetic powder selected from the group consisting of talc, nylon powder, lauroyl lysine, zinc oxide, titanium dioxide, iron oxides, and combinations thereof; (b) from about 0.1% to about 1.5% by weight of a thermoplastic elastomer comprising a styrene isobutylene styrene (SIBS) block copolymer; (c) from about 1% to about 20% by weight of a non-volatile oil; and (d) from about 0.01% to about 5% by weight of a film-forming polymer comprising PEG-12/SMDI Copolymer. The compositions are in the form of a powder, for example a pressed powder or loose powder, and desirably exhibit longer wear on human skin than an otherwise identical composition that does not include components (b)-(d).

In another aspect of the invention, a method is provided for imparting a long-wearing powder to the skin, comprising topically applying to skin any of the compositions according to the invention.

### DESCRIPTION OF THE INVENTION

The present invention provides novel powder-based cosmetics that have excellent wear attributes. By long-wearing is meant that the compositions are less prone to transfer from a human integument to a substrate on contact therewith than traditional powder-based products. For the purposes of this invention, the term "integument" includes, without limitation, skin of the face, skin of the body, lips, eyelids, eyelashes, and hair of the scalp.

The powder-based compositions according to the instant invention comprise a thermoplastic elastomer. In the broadest aspect of the invention, the thermoplastic elastomer may be any such polymer that is safe for topical application to a human integument. Generally, thermoplastic elastomers are polymers which have hard segments and soft segments. Thermoplastic elastomers may be block copolymers, for example, diblock, triblock, and multi-block copolymers. The thermoplastic elastomers useful in the invention are typically A-B di-block, A-B-A tri-block, (A-B)ₙ multi-block, and (A-B)ₙₓ (where x represents an n functional joint) copolymers, where A represents a hard domain (high T_{g}) and B represents a soft domain (low Tg).

Representative copolymers include those where the hard segments comprise polystyrene units and the soft segments are polyolefins. Styrene-isobutylene-styrene (SIBS), styrene-butadiene-styrene, styrene-isoprene-styrene, or styrene-ethylenebutylene-styrene are examples. Styrene-isobutylene-styrene (SIBS) is the preferred thermoplastic elastomer according to the invention. Heretofore, this polymer has found only limited use in cosmetics because it is insoluble in most cosmetic solvents and therefore is extremely difficult to formulate.

The compositions may comprise from about 0.01% to about 10% by weight of the SIBS polymer, but will more typically comprise from about 0.1% to about 5% by weight SIBS. Preferably, the SIBS polymer will comprise from about 0.5 to about 1.5% by weight, and more preferred still, about 1% by weight of the composition.

The inventive compositions will also include a non-volatile oil. By non-volatile is meant that the oil does not readily evaporate at room temperature under atmospheric pressure. The non-volatile oils may be, without limitation, hydrocarbons, ester oils, particularly fatty acid esters, or silicones.

Ester oils include any non-polar or low-polarity esters, including fatty acid esters. Special mention may be made of those esters commonly used as emollients in cosmetic formulations. Such esters will typically be the esterification product of an acid of the form R₄(COOH)₁₋₂ with an alcohol of the form R₅(OH)₁₋₃ where R₄ and R₅ are each independently linear, branched, or cyclic hydrocarbon groups, optionally containing unsaturated bonds, and having from 1 to 30 carbon atoms, preferably from 2 to 30 carbon atoms, and more preferably, from 3 to 30 carbon atoms. Preferably, at least one of R₄ and R₅ comprises at least 10, and more preferably, at least 15 carbon atoms, such that the ester comprises at least one fatty chain. The esters defined above will include, without limitation, the esters of mono-acids with mono-alcohols, mono-acids with diols and triols, di-acids with mono-alcohols, and tri-acids with mono-alcohols.

Suitable fatty acid esters include, without limitation, butyl acetate, butyl isostearate, butyl oleate, butyl octyl oleate, cetyl palmitate, ceyl octanoate, cetyl laurate, cetyl lactate, cetyl isononanoate, cetyl stearate, diisostearyl fumarate, diisostearyl malate, neopentyl glycol dioctanoate, dibutyl sebacate, di-C₁₂₋₁₃ alkyl malate, dicetearyl dimer dilinoleate, dicetyl adipate, diisocetyl adipate, diisononyl adipate, diisopropyl dimerate, triisostearyl trilinoleate, octodecyl stearoyl stearate, hexyl laurate, hexadecyl isostearate, hexydecyl laurate, hexyldecyl octanoate, hexyldecyl oleate, hexyldecyl palmitate, ethyhexyl palmitate, octyl palmitate, hexyldecyl stearate, isononyl isononanaote, isostearyl isononate, isohexyl neopentanoate, isohexadecyl stearate, isopropyl isostearate, n-propyl myristate, isopropyl myristate, n-propyl palmitate, isopropyl palmitate, hexacosanyl palmitate, lauryl lactate, octacosanyl palmitate, propylene glycol monolaurate, triacontanyl palmitate, dotriacontanyl palmitate, tetratriacontanyl palmitate, hexacosanyl stearate, octacosanyl stearate, triacontanyl stearate, dotriacontanyl stearate, stearyl lactate, stearyl octanoate, stearyl heptanoate, stearyl stearate, tetratriacontanyl stearate, triarachidin, tributyl citrate, triisostearyl citrate, tri-C₁₂₋₁₃-alkyl citrate, tricaprylin, tricaprylyl citrate, tridecyl behenate, trioctyldodecyl citrate, tridecyl cocoate, tridecyl isononanoate, glyceryl monoricinoleate, 2-octyldecyl palmitate, 2-octyldodecyl myristate or lactate, di(2-ethylhexyl) succinate, tocopheryl acetate, and the like.

Salicylates and benzoates are also contemplated to be useful esters in the practice of the invention. Suitable salicylates and benzoates include esters of salicylic acid or benzoic acid with an alcohol of the form R₆OH where R₆ is a linear, branched, or cyclic hydrocarbon group, optionally containing unsaturated bonds, and having from 1 to 30 carbon atoms, preferably from 6 to 22 carbon atoms, and more preferably from 12 to 16 carbon atoms. Suitable salicylates include, for example, octyl salicylate and hexyldodecyl salicylate, and benzoate esters including C₁₂₋₁₅ alkyl benzoate, isostearyl benzoate, hexyldecyl benzoate, benzyl benzoate, and the like.

The oil may also be a volatile or non-volatile silicone oil. Suitable silicone oils include linear or cyclic silicones such as polyalkyl- or polyarylsiloxanes, optionally comprising alkyl or alkoxy groups having from 1 to 10 carbon atoms. Representative silicone oils include, for example, caprylyl methicone, cyclomethicone, cyclopentasiloxane decamethylcyclopentasiloxane, decamethyltetrasiloxane, diphenyl dimethicone, dodecamethylcyclohexasiloxane, dodecamethylpentasiloxane, heptamethylhexyltrisiloxane, heptamethyloctyltrisiloxane, hexamethyldisiloxane, methicone, methyl-phenyl polysiloxane, octamethylcyclotetrasiloxane, octamethyltrisiloxane, perfluorononyl dimethicone, polydimethylsiloxanes, and combinations thereof. In one embodiment, the silicone oil will be essentially free of cyclomethicones, by which is meant that no cyclomethicones are intentionally added.

The silicone oil will typically, but not necessarily, have a viscosity of between about 5 and about 3,000 centistokes (cSt), preferably between 50 and 1,000 cSt measured at 25°C.

In one embodiment, the silicone oil comprises phenyl groups, as is the case with methylphenylpolysiloxane, INCI name diphenyl dimethicone, commercially available from Shin Etsu Chemical Co under a variety of tradenames including F-5W, KF-54 and KF-56. Diphenyl dimethicones have good organic compatibility and impart film-forming characteristics to the product. Further, the presence of phenyl groups increases the refractive index of the silicone oil, contributing high gloss to the product if desired. Another suitable phenyl-functionalized silicone oil has the INCI name phenyltrimethicone and is sold under the trade name "DC 556" by Dow Corning.

In one embodiment of the invention, the silicone oil may comprise a fluorinated silicone, such as a perfluorinated silicone (i.e., fluorosilicones). Fluorosilicones are advantageously both hydrophobic and oleophobic and thus impart desirable tactile aesthetics to the product. Fluorosilicones also may impart long-wearing characteristics. The preferred fluorosilicone is a fluorinated organofunctional silicone fluid having the INCI name perfluorononyl dimethicone. Perfluorononyl dimethicone is commercially available from Pheonix Chemical under the trade name Pecosil®.

The compositions may also comprise hydrocarbon oils. Exemplary hydrocarbon oils are straight or branched chain paraffinic hydrocarbons having from 5 to 80 carbon atoms, preferably from 8 to 40 carbon atoms, and more preferably from 10 to 16 carbon atoms, including but not limited to, pentane, hexane, heptane, octane, nonane, decane, undecane, dodecane, tetradecane, tridecane, and the like. Preferred hydrocarbon oils are highly branched aliphatic hydrocarbons, including C₈₋₉ isoparaffins, C₉₋₁₁ isoparaffins, C₁₂ isoparaffin, and C₂₀₋₄₀ isoparaffins and the like. Special mention may be made of the isoparaffins having the INCI names isohexadecane, isoeicosane, and isododecane.

Also suitable as hydrocarbon oils are polyalphaolefins, typically having greater than 20 carbon atoms, including C₂₄₋₂₈ olefins, C₃₀₋₄₅ olefins, hydrogenated polyisobutene, hydrogenated polydecene, polybutene, mineral oil, pentahydrosqualene, squalene, squalane, and the like. The hydrocarbon oil may also comprise higher fatty alcohols, such as oleyl alcohol, octyldodecanol, and the like.

Other suitable oils include without limitation castor oil, C₁₀₋₁₈ triglycerides, caprylic/capric/triglycerides, coconut oil, corn oil, cottonseed oil, linseed oil, mink oil, olive oil, palm oil, illipe butter, rapeseed oil, soybean oil, sunflower seed oil, walnut oil, avocado oil, camellia oil, macadamia nut oil, turtle oil, mink oil, soybean oil, grape seed oil, sesame oil, maize oil, rapeseed oil, sunflower oil, cottonseed oil, jojoba oil, peanut oil, olive oil, and combinations thereof.

Any one of the foregoing ester oils, silicone oils, and hydrocarbon oils are contemplated to be useful in the practice of the invention. Accordingly, in one embodiment, the compositions comprise at least one oil selected from the ester oils, silicone oils, and hydrocarbon oils described above. In another embodiment, the compositions comprise two or more oils selected from the ester oils, silicone oils, and hydrocarbon oils described above. Because the ester oils described herein function as emollients, it is preferred that the compositions comprise at least one ester oil, and will optionally comprise at least one additional oil selected from hydrocarbon oils, silicone oils, and combinations thereof. In one embodiment, the compositions will comprise at least one ester and at least one silicone oil. In another embodiment, the compositions will comprise isopropyl palmitate in combination with a silicone fluid.

The oils will collectively comprise from about 0.1% to about 45% by weight of the compositions, but will typically comprise from about 0.5% to about 25% by weight, and more typically from about 1% to about 20% by weight of the composition. In a preferred embodiment, the oils will collectively comprise from about 5% to about 15% by weight of the composition.

The film forming polymer (or "film former") contemplated in accordance with the present invention may improve smoothness or spreadability, water-resistance, transfer resistance properties, or other cosmetic or pharmaceutical properties desired by one of skill in the art. The term film-forming polymer may be understood to indicate a polymer which is capable, by itself or in the presence of at least one auxiliary film-forming agent, of forming a continuous film which adheres to a surface and functions as a binder for the particulate material. It is preferable hydrophobic and compatible with the thermoplastic elastomer, including SIBS.

The film-forming polymer will preferably comprise a polypropylene glycol (PPG) backbone present in an amount of about 0.1% to about 1% by weight of the total composition. In one embodiment, the film-forming polymer is a polyurethane polymer formed by the reaction of a polypropylene glycol diol and a diisocyanate. The polypropylene glycol diol may have the structure: 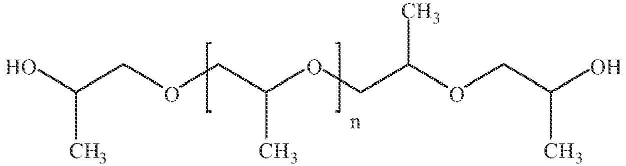 where "n" is an integer from 1 to 100. Typically, "n" will be an integer from 2 to 50, and more typically "n" is an integer from 5 to 20.

The diisocyanate will be of the form O=C=N-R^{d}-N=C=O, where R^{d} is a divalent hydrocarbon group containing from 1 to 20 carbon atoms, including optional substitution with one or more heteroatoms, and in particular R^{d} will be selected from optionally substituted, branched, straight chain, or cyclic alkyl, alkenyl, alkynyl, aryl, alkylaryl, or aryl-alkyl groups; including without limitation:
(i) a group of the form: 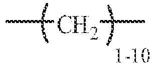
(ii) a group of the form: 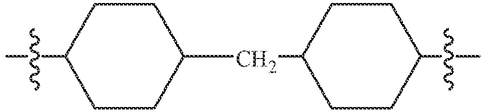
(ii) a group of the form: 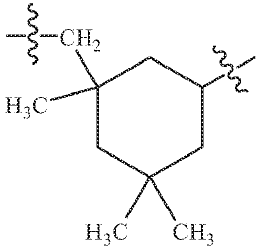
(iv) a group of the form: 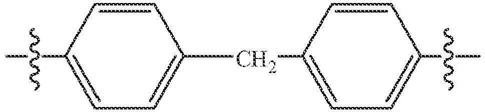 and;
(v) a group of the form: 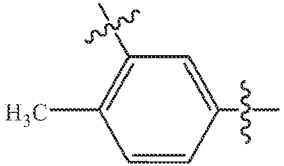
and combinations thereof.

Suitable diisocyanates include, without limitation, toluene diisocyanate; methylene diphenyl diisocyanate, including 2,2'-MDI, 2,4'-MDI, and 4,4'-MDI; 1,6-hexamethylene diisocyanate; isophorone diisocyanate; methylene dicyclohexyl diisocyanate; xylene diisocyanate; cyclohexane diisocyanate; 3,3'-dimethyl-4,4'-diphenylmethane diisocyanate; p-phenylene diisocyanate; m-phenylene diisocyanate; 4,4'-isopropylidene dicyclohexyl isocyanate; and the like. In a preferred embodiment, the diisocyanate is methylene dicyclohexyl diisocyanate, also known as methylenebis(4-cyclohexyl isocyanate) (SMDI).

The preferred film former is a polyurethane formed by the reaction of a polypropylene glycol with methylenebis(4-cyclohexyl isocyanate) and has the CTFA name PEG-12/SMDI Copolymer (INCI).

The preferred concentration of the film forming polymer comprising a polypropylene glycol backbone will typically be used in an amount from 0.01% to 20% by weight, relative to the total weight of the composition. More typically, the film forming polymer comprising a polypropylene glycol backbone will be present in an amount from about 0.05% to about 10% by weight, and preferably from about 0.1% to about 5% by weight, and more preferred still, from about 0.1% to about 1% by weight of the entire composition.

The composition may optionally include one or more additional film forming polymers in an amount from about 0.001% to about 10% by weight. Polymeric film formers include cellulosics, polyolefins, polyvinyls, polyacrylates, polyurethanes, silicones, silicone acrylates, polyamides, polyesters, fluoropolymers, polyethers, polyacetates, polycarbonates, polyimides, epoxys, formaldehyde resins, and homopolymers and copolymers of any of the foregoing.

The compositions may optionally include a wax component. The wax component may be a single wax or may comprise a combination of waxes. Any wax compatible with a cosmetic product is contemplated to be suitable, including without limitation natural, mineral and/or synthetic waxes. Natural waxes are those of animal origin, including without limitation beeswax, spermaceti, lanolin, and shellac wax, and those of vegetable origin, including without limitation carnauba, candelilla, bayberry, sugarcane wax, and the like. Natural waxes may also include polycosanols. Mineral waxes contemplated to be useful include, without limitation ozokerite, ceresin, montan, paraffin, microcrystalline, petroleum, and petrolatum waxes. Synthetic waxes include, for example, Fischer Tropsch (FT) waxes and polyolefin waxes, such as ethylene homopolymers, ethylene-propylene copolymers, and ethylene-hexene copolymers. Representative ethylene homopolymer waxes are commercially available under the tradename POLYWAX^{®} Polyethylene (Baker Hughes Incorporated). Commercially available ethylene-α-olefin copolymer waxes include those sold under the tradename PETROLITE^{®} Copolymers (Baker Hughes Incorporated). Synthetic waxes also include, for example, polyethylene glycols such as PEG-18, PEG-20, PEG-32, PEG-75, PEG-90, PEG-100, and PEG-180 which are sold under the tradename Carbowax^{®} (The Dow Chemical Company). Other suitable waxes include silicone wax, microcrystalline wax, polyethylene wax, or a polydimethylsiloxane with a high molecular weight hydrocarbonalkyl, such as the wax having the INCI name C₃₀₋₄₅ alkyl methicone (and) C₃₀₋₄₅ olefins. In one embodiment, the compositions are free of wax or are essentially free of wax, by which is meant that wax components collectively comprise less than 0.1% of the composition.

The composition of the instant invention are powder-based and therefore comprise from at least about 30% up to about 98% by weight powdered material. The powdered materials are any powdered materials useful in cosmetic or personal care products. The powders may comprise any shape and size particles including, for example, spherical, amorphous, and platelet shaped particles. The powders will typically have a median particle size greater than 0.01 microns and less than 300 microns, but more typically will range from about 0.1 microns to about 150 microns, and preferably from about 1 micron to about 75 microns.

The cosmetic powders will typically comprise fillers, pigments, and pearls. Suitable fillers include without limitation silica, surface treated silica, alumina, surface-treated alumina, talc and surface treated talc, zinc stearate, mica and surface treated mica, kaolin, Nylon powders such as Orgasol™, polyethylene powder, Teflon™, starch, boron nitride, Lauroyl Lysine, copolymer microspheres such as Expancel™ (Nobel Industries), cross-linked polymethacrylate copolymers such as Polytrap™ (Dow Corning), and silicone resin microbeads (Tospearl™ from Toshiba), and the like.

The preferred filler is mica, preferably in the form of sericite. Sericite may comprise from about 5% to about 98% by weight of the composition, more typically from about 10% to about 85% by weight, and preferably from about 20% to about 75% by weight of the composition, and more preferred still from about 30% to about 65% of the composition. In preferred implementations, the compositions comprise sericite in combination with one or more fillers selected from the group consisting of talc, Nylon powder, Lauroyl Lysine, and kaolin. In one embodiment, the composition comprises sericite, talc (preferably chitosan-treated talc), Nylon powder, Lauroyl Lysine, and kaolin.

Additional powder fillers include, but are not limited to, inorganic powders such as gums, chalk, calcium oxide, calcium carbonate, magnesium oxide, magnesium carbonate, Fuller's earth, attapulgite, bentonite, muscovite, phlogopite, synthetic mica, lepidolite, hectorite, biotite, lithia mica, vermiculite, aluminum silicate, aluminum magnesium silicate, diatomaceous earth, starch, alkyl and/or trialkyl aryl ammonium smectites, chemically modified magnesium aluminum silicate, organically modified montmorillonite clay, hydrated aluminum silicate, hydrated silica, fumed aluminum starch octenyl succinate barium silicate, calcium silicate, magnesium silicate, strontium silicate, metal tungstate, magnesium, silica alumina, zeolite, barium sulfate, calcined calcium sulfate (calcined gypsum), calcium phosphate, fluorine apatite, hydroxyapatite, ceramic powder, metallic soap (zinc stearate, magnesium stearate, zinc myristate, calcium palmitate, and aluminum stearate), colloidal silicon dioxide; organic powder, cyclodextrin, methyl polymethacrylate powder, copolymer powder of styrene and acrylic acid, benzoguanamine resin powder, poly(ethylene tetrafluoride) powder, and carboxyvinyl polymer, cellulose powder such as hydroxyethyl cellulose and sodium carboxymethyl cellulose, and ethylene glycol monostearate. Other useful powders are known to one skilled in the art and include those disclosed in U.S. Pat. No. 5,688,831.

The compositions of the instant invention will typically include one or more pigments. Suitable pigments are well known in the art and include those disclosed in the C.T.F.A. Cosmetic Ingredient Handbook, First Edition, 1988. Exemplary pigments include, but are not limited to, metal oxides and metal hydroxides such as magnesium oxide, magnesium hydroxide, calcium oxide, calcium hydroxides, aluminum oxide, aluminum hydroxide, iron oxides (α-Fe₂O₃, β-Fe₂O₃, Fe₃O₄, FeO), red iron oxide, yellow iron oxide, black iron oxide, iron hydroxides, titanium dioxide, titanium lower oxides, zirconium oxides, chromium oxides, chromium hydroxides, manganese oxides, cobalt oxides, cerium oxides, nickel oxides and zinc oxides and composite oxides and composite hydroxides such as iron titanate, cobalt titanate and cobalt aluminate. Other suitable pigments include ultramarine blue (*i.e*., sodium aluminum silicate containing sulfur), Prussian blue, manganese violet and the like.

Suitable pearling (nacreous) pigments include without limitation bismuth oxychloride, guanine, and titanium composite materials containing, as a titanium component, titanium dioxide, titanium lower oxides or titanium oxynitride, as disclosed in U.S. Patent No. 5,340,569. Preferred pearls include titanated micas which may be white or colored by the additional inclusion of iron oxides on the mica. The compositions may also include glittering agents.

The fillers, pigments, and pearls may be surface modified to adjust one or more characteristics of the particle, including its affinity for the skin, hydrophobicity, hydrophilicity, etc. The surface treatment may form a coating on the particles and may by covalently bound to the particle. Representative coating materials include alkanes, siloxanes, fluoroalkanes, amino acids and peptides, and carbohydrate polymers. Hydrophobically modified particulates and methods for preparing hydrophobically modified particulates are described in, for example, U.S. Patent No. 3,393,155 to Schutte et al., U.S. Patent No. 2,705,206 to Wagner et al., U.S. Patent No. 5,500,216 to Wagner et al., U.S. Patent No. 6,683,126 to Keller et al., and U.S. Patent No. 7,083,828 to Müller et al., U.S. Patent Pub. No. 2006/0110541 to Russell at al., and U.S. Patent Pub. No. 2006/0110542 to Dietz et al. A preferred coating is methicone. Another preferred hydrophobic coating according to the invention is prepared by treating an oxide having reactive -OH groups with Trimethoxycaprylyl Silane.

In one embodiment, the compositions will comprise mica that has been treated with Lauroyl Lysine, talc that has been treated with chitosan, titanium dioxide that has been treated with methicone, zinc oxide that has been treated with dimethicone, and/or titanium dioxide that has been treated with Trimethoxycaprylyl Silane. The compositions will also typically include surface treated iron oxides.

In some embodiments, the powders include materials that impart SPF to the product, including titanium dioxide and/or zinc oxide. The titanium dioxide may be substantially or entirely anatase or rutile. Titanium dioxide may comprise, for example, from about 1% to about 20% by weight of the composition, more typically from about 2% to about 15% by weight. Zinc oxide may comprise, for example, from about 1% to about 20% by weight of the composition, more typically from about 2% to about 15% by weight.

The compositions may further include a dry powder binder such as zinc stearate. When present, zinc stearate will typically comprise from about 0.01% to about 2% by weight of the composition, more typically from about 0.05% to about 1.25% by weight.

The compositions may take the form of a face foundation, concealer, blush, pressed powder, loose powder, mosaic powder, two-in-one powder, multi color powder, extruded powder, extruded powder bead, baked powder, baked powder beads, single or multicolor cream beads, wax beads, extruded face powder, eye shadow, powder blush, wet dry powder foundation, body talc powder, fragrance talc powder, or other powder-based cosmetic or personal care product.

In other embodiments, the compositions of the invention may be chosen from molded and poured cosmetics and cosmetic sticks. For example, forms in which the compositions of the invention may exist include eyeshadow, eyeshadow base, blush, lipstick, fragrance delivery system, deodorant stick, skin protectant stick, sunscreen stick, medication cream or other medication application system, adhesive or insect repellent. In another embodiment, the composition is a base for a cosmetic powder. For example, the composition may be applied to the skin as a base and the cosmetic powder is then applied to the base. The composition of the instant invention may also be in the form of a long wearing pressed powder-type colorless cream/lotion that can hold/retain skin moisture for long periods of time thus providing long lasting moisturization.

The composition may also be in the form of a pressed powder composition in which the composition contains various colorants, for example lakes, pigments, pearlescents, and combinations thereof. Especially useful are pressed powder compositions in which the composition contains a C₁₂₋₁₅ alkyl benzoate. It has been found that this material advantageously helps maintain the integrity of the pressed powder at elevated temperatures as may be encountered during warehouse storage conditions in some locations, e.g., 95 to 125 °F.

In addition, the compositions may additionally include any additive usually employed in the field envisaged such as dyes, antioxidants, perfumes, essential oils, stabilizers, moisturizers, vitamins, sunscreens, emollients, humectants, thickeners, clays, gums, plasticizers, gellants, chelating agents, pH adjusters, preservatives, solvents, surfactants, spreading agents, dispersants, preservatives, antifoaming agents, wetting agents, ultraviolet-screening agents, cosmetic or pharmaceutical active agents, moisturizers, vitamins and derivatives thereof, and botanicals.

The present invention in another embodiment is directed to a method of imparting the long-wearing powder-based composition of the instant invention to a keratinous surface such as the skin or face. The powder-based composition is ideally long-wearing and resistant to transfer. In various embodiments, the composition will maintain a freshly applied look for at least one hour, and preferably at least two hours, three hours, six hours, or even more. The composition will have greater substantivity to the skin and hence longer wear than otherwise identical compositions that do not include the SIBS and/or polypropylene glycol backbone polymeric film former.

### EXAMPLE 1

A long-wearing pressed-powder color cosmetic is provided in Table 1.

**Table 1.**

| Ingredient | % by Weight | % by Weight |
|---|---|---|
| PART A | | |
| (Dry Powders) | | |
| Mica | qs | qs |
| Nylon Powder | 6 | 6 |
| Talc | 5 | 5 |
| Lauroyl Lysine | 3 | 3 |
| Zinc Stearate | 1.25 | 1.25 |
| Kaolin | 0.25 | 0.25 |
| Titanium Dioxide | 8 | 9.5 |
| Zinc Oxide | 7 | 7 |
| Iron Oxides | 0.2 | 1.1 |
| Preservatives | 0.5 | 1 |
| Chelating agent | 0.2 | - |

| PART B | | |
|---|---|---|
| (Oil Phase) | | |
| Silicone Fluid | 1 | 1 |
| PPG-12/SMDI Copolymer | 0.5 | 0.5 |
| C₁₂₋₁₅ Alcohols Benzoate | 7. | 9 |
| Isobutylene/Styrene Copolymer | 1 | 1 |

Part A is mixed in a powder mixer along with colors. Once a uniform powder phase is achieved then the entire mixture is passed through a jet mill to disperse color completely and uniformly. The resulting uniform dry powder is put back into the powder mixer and the uniformly melted hot oil phase Part B is sprayed onto the jet milled powder. The entire combined phase is then passed through a micronizer and the final bulk is pressed into a pan.

All percentages are by weight, based on the total weight of the composition, unless otherwise indicated.

## Claims

1. A long-wearing powder-based cosmetic composition comprising:
a. from about 75% to about 95% by weight of one or more powdered materials useful in cosmetics;
b. from about 0.01% to about 5% by weight of a thermoplastic elastomer comprising a styrene isobutylene styrene (SIBS) block copolymer;
c. from about 1% to about 20% by weight of a non-volatile oil; and
d. from about 0.01% to about 5% by weight of a film-forming polymer comprising a polypropylene glycol (PPG) backbone;
wherein the composition is in the form of a powder which exhibits longer wear on human skin than an otherwise identical composition that does not include said SIBS block copolymer, non-volatile oil, which does not readily evaporate at room temperature and atmospheric pressure and film-forming polymer comprising a polypropylene glycol (PPG) backbone.

2. The composition according to claim 1, wherein said SIBS block copolymer is present in an amount of about 0.1% to about 1.5% by weight of the total composition, and wherein said film-forming polymer comprising a polypropylene glycol (PPG) backbone is a polyurethane polymer formed by the reaction of a polypropylene glycol diol and a diisocyanate present in an amount of about 0.1% to about 1% by weight of the total composition.

3. The composition according to claims 1 or 2, wherein said polypropylene glycol diol has the structure: 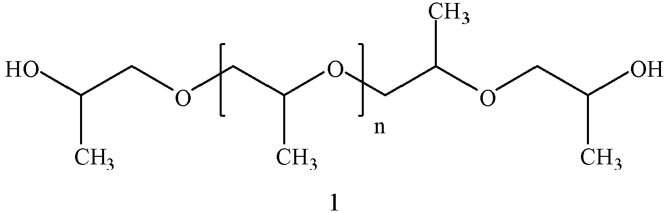 where "n" is an integer from 1 to 100.

4. The composition according to claims 1-3, wherein "n" is an integer from 5 to 20.

5. The composition according to claims1-4, wherein said diisocyante is methylenebis(4-cyclohexyl isocyanate) (SMDI), wherein said non-volatile oil comprises an ester oil, and wherein said non-volatile oil comprises a silicone oil..

6. The composition of claims 1-5, wherein the long wearing powder-based cosmetic composition is in the form of a pressed powder.

7. The composition of claims 1-6, wherein the non-volatile oil comprises a C₁₂₋₁₅ alkyl benzoate or a mixture of a silicone oil and a C₁₂₋₁₅ alkyl benzoate..

8. The composition of claim 1-7 a long-wearing powder-based cosmetic composition, wherin
a. one or more of the cosmetic powders comprising sericite and at least one other cosmetic powder selected from the group consisting of talc, nylon powder, lauroyl lysine, zinc oxide, titanium dioxide, iron oxides, and combinations thereof;
b. the thermoplastic elastomer comprising a styrene isobutylene styrene (SIBS) block copolymer;
c. the film-forming polymer comprising PEG-12/SMDI Copolymer;
wherein the composition is in the form of a powder which exhibits longer wear on human skin than an otherwise identical composition that does not include said SIBS block copolymer, non-volatile oil, which does not readily evaporate at room temperature and under atmospheric pressure and PEG-12/SMDI Copolymer.

9. The composition of claim 8, wherein the long wearing powder-based cosmetic composition is in the form of a pressed powder.

10. The composition of claim 15, wherein the non-volatile oil comprises a C₁₂₋₁₅ alkyl benzoate or a mixture of a silicone oil and a C₁₂₋₁₅ alkyl benzoate.

11. A method of imparting long-wearing and transfer-resistant powder-based cosmetic to the skin comprising applying to the skin a composition comprising:
a. from about 75% to about 95% by weight of one or more powder-based cosmetics;
b. from about 0.01% to about 5% by weight of a thermoplastic elastomer comprising a styrene isobutylene styrene (SIBS) block copolymer;
c. from about 1% to about 20% by weight of a non-volatile oil; and
d. from about 0.01% to about 5% by weight of a film-forming polymer comprising a polypropylene glycol (PPG) backbone;
wherein the composition is in the form of a powder which exhibits longer wear on human skin than an otherwise identical composition that does not include said SIBS block copolymer, non-volatile oil, and film-forming polymer comprising a polypropylene glycol (PPG) backbone.

12. The method according to claim 11, wherein said film-forming polymer comprising a polypropylene glycol (PPG) backbone is a polyurethane polymer formed by the reaction of a polypropylene glycol diol and a diisocyanate, wherein said polypropylene glycol diol has the structure: 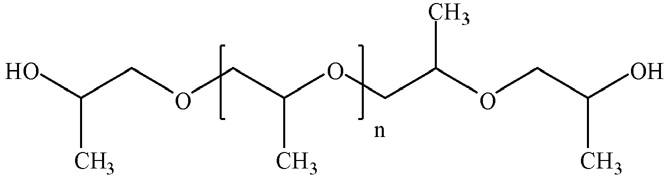 where "n" is an integer from 1 to 100, and wherein said diisocyante is methylenebis(4-cyclohexyl isocyanate) (SMDI).

13. The method according to claims 11-12, wherein said non-volatile oil comprises an ester oil, and wherein said non-volatile oil comprises a silicone oil.

14. The method according to claims 11-13, wherein the long wearing powder-based cosmetic composition is in the form of a pressed powder.

15. The method of claims 11-14, wherein the non-volatile oil comprises a C₁₂₋₁₅ alkyl benzoate or a mixture of a silicone oil and a C₁₂₋₁₅ alkyl benzoate..

## Patentansprüche

1. Lange haltende kosmetische Zusammensetzung auf Pulverbasis, umfassend:
a. zu etwa 75 Gew.-% bis etwa 95 Gew.-% eine oder mehrere pulverisierte Substanzen, die in Kosmetika brauchbar sind;
b. zu etwa 0,01 Gew.-% bis etwa 5 Gew.-% ein thermoplastisches Elastomer, das ein Styrol-Isobutylen-Styrol(SIBS)-Blockcopolymer umfasst;
c. zu etwa 1 Gew.-% bis etwa 20 Gew.-% ein nichtflüchtiges Öl; und
d. zu etwa 0,01 Gew.-% bis etwa 5 Gew.-% ein filmbildendes Polymer, das ein Polypropylenglycol(PPG)-Rückgrat enthält;
wobei die Zusammensetzung in Form eines Pulvers vorliegt, das eine längere Haltbarkeit auf menschlicher Haut zeigt als eine ansonsten identische Zusammensetzung, die das SIBS-Blockcopolymer, das nichtflüchtige Öl, welches bei Raumtemperatur und unter Atmosphärendruck nicht leicht verdampft, und das filmbildende Polymer mit einem Polypropylenglycol(PPG)-Rückgrat nicht enthält.

2. Zusammensetzung nach Anspruch 1, wobei das SIBS-Blockcopolymer in einer Menge von etwa 0,1 Gew.-% bis etwa 1,5 Gew.-% der Gesamtzusammensetzung vorliegt und wobei das filmbildende Polymer mit einem Polypropylenglycol(PPG)-Rückgrat ein Polyurethan-Polymer ist, das durch die Reaktion eines Polypropylenglycol-Diols und eines Diisocyanats gebildet wird und in einer Menge von etwa 0,1 Gew.-% bis etwa 1 Gew.-% der Gesamtzusammensetzung vorliegt.

3. Zusammensetzung nach Anspruch 1 oder 2, wobei das Polypropylenglycol-Diol die Struktur: 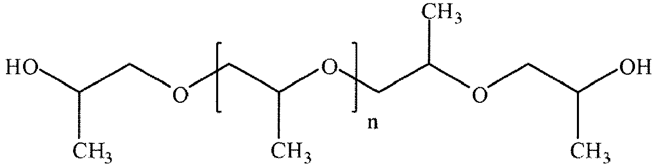 aufweist, wobei "n" eine ganze Zahl von 1 bis 100 ist.

4. Zusammensetzung nach Anspruch 1 bis 3, wobei "n" eine ganze Zahl von 5 bis 20 ist.

5. Zusammensetzung nach Anspruch 1 bis 4, wobei das Diisocyanat Methylenbis(4-cyclohexyl-isocyanat) (SMDI) ist, wobei das nichtflüchtige Öl ein Ester-Öl umfasst und wobei das nichtflüchtige Öl ein Silikonöl umfasst.

6. Zusammensetzung von Anspruch 1 bis 5, wobei die lange haltende kosmetische Zusammensetzung auf Pulverbasis in Form eines gepressten Pulvers vorliegt.

7. Zusammensetzung von Anspruch 1 bis 6, wobei das nichtflüchtige Öl ein C₁₂₋₁₅-Alkylbenzoat oder ein Gemisch aus einem Silikonöl und einem C₁₂₋₁₅-Alkylbenzoat umfasst.

8. Zusammensetzung von Anspruch 1 bis 7, eine lange haltende kosmetische Zusammensetzung auf Pulverbasis, wobei
a. eines oder mehrere der kosmetischen Pulver Serizit und wenigstens ein weiteres kosmetisches Pulver umfassen, das aus der Gruppe bestehend aus Talkum, Nylonpulver, Lauroyllysin, Zinkoxid, Titandioxid, Eisenoxiden und Kombinationen davon ausgewählt ist;
b. das thermoplastische Elastomer ein Styrol-Isobutylen-Styrol(SIBS)-Blockcopolymer umfasst;
c. das filmbildende Polymer PEG-12/SMDI-Copolymer umfasst;
wobei die Zusammensetzung in Form eines Pulvers vorliegt, das eine längere Haltbarkeit auf menschlicher Haut zeigt als eine ansonsten identische Zusammensetzung, die das SIBS-Blockcopolymer, das nichtflüchtige Öl, welches bei Raumtemperatur und unter Atmosphärendruck nicht leicht verdampft, und das PEG-12/SMDI-Copolymer nicht enthält.

9. Zusammensetzung von Anspruch 8, wobei die lange haltende kosmetische Zusammensetzung auf Pulverbasis in Form eines gepressten Pulvers vorliegt.

10. Zusammensetzung von Anspruch 15, wobei das nichtflüchtige Öl ein C₁₂₋₁₅-Alkylbenzoat oder ein Gemisch aus einem Silikonöl und einem C₁₂₋₁₅-Alkyl-benzoat umfasst.

11. Verfahren zur Verabreichung eines lange haltenden und transferbeständigen Kosmetikums auf Pulverbasis an die Haut, das die Applikation einer Zusammensetzung auf die Haut beinhaltet, welche umfasst:
a. zu etwa 75 Gew.-% bis etwa 95 Gew.-% ein oder mehrere Kosmetika auf Pulverbasis;
b zu etwa 0,01 Gew.-% bis etwa 5 Gew.-% ein thermoplastisches Elastomer, das ein Styrol-Isobutylen-Styrol(SIBS)-Blockcopolymer umfasst;
c. zu etwa 1 Gew.-% bis etwa 20 Gew.-% ein nichtflüchtiges Öl; und
d. zu etwa 0,01 Gew.-% bis etwa 5 Gew.-% ein filmbildendes Polymer, das ein Polypropylenglycol(PPG)-Rückgrat enthält;
wobei die Zusammensetzung in Form eines Pulvers vorliegt, das eine längere Haltbarkeit auf menschlicher Haut zeigt als eine ansonsten identische Zusammensetzung, die das SIBS-Blockcopolymer, das nichtflüchtige Öl und das filmbildende Polymer mit einem Polypropylenglycol(PPG)-Rückgrat nicht enthält.

12. Verfahren nach Anspruch 11, wobei das filmbildende Polymer mit einem Polypropylenglycol(PPG)-Rückgrat ein Polyurethan-Polymer ist, das durch die Reaktion eines Polypropylenglycol-Diols und eines Diisocyanats gebildet wird, wobei das Polypropylenglycol-Diol die Struktur: 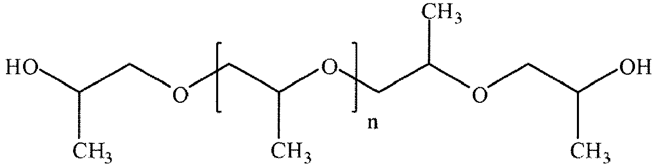 aufweist, wobei "n" eine ganze Zahl von 1 bis 100 ist, und wobei das Diisocyanat Methylenbis(4-cyclohexyl-isocyanat) (SMDI) ist.

13. Verfahren nach Anspruch 11 bis 12, wobei das nichtflüchtige Öl ein Ester-Öl umfasst und wobei das nichtflüchtige Öl ein Silikonöl umfasst.

14. Verfahren nach Anspruch 11 bis 13, wobei die lange haltende kosmetische Zusammensetzung auf Pulverbasis in Form eines gepressten Pulvers vorliegt.

15. Verfahren nach Anspruch 11 bis 14, wobei das nichtflüchtige Öl ein C₁₂₋₁₅-Alkylbenzoat oder ein Gemisch aus einem Silikonöl und einem C₁₂₋₁₅-Alkyl-benzoat umfasst.

## Revendications

1. Composition cosmétique à base de poudre longue tenue comprenant :
a. d'environ 75 % à environ 95 % en poids d'une ou plusieurs matières en poudre utiles en cosmétique ;
b. d'environ 0,01 % à environ 5 % en poids d'un élastomère thermoplastique comprenant un copolymère bloc de styrène-isobutylène-styrène (SIBS) ;
c. d'environ 1 % à environ 20 % en poids d'une huile non volatile ; et
d. d'environ 0,01 % à environ 5 % en poids d'un polymère filmogène comprenant un squelette de polypropylène-glycol (PPG) ;
laquelle la composition se présente sous la forme d'une poudre qui présente une tenue plus longue sur la peau humaine qu'une composition identique par ailleurs, qui n'inclut pas ledit copolymère bloc SIBS, ladite huile non volatile qui ne s'évapore pas facilement à la température de la pièce et à la pression atmosphérique ni ledit polymère filmogène comprenant un squelette de polypropylène-glycol (PPG).

2. Composition selon la revendication 1, dans laquelle ledit copolymère bloc SIBS est présent dans une quantité d'environ 0,1 % à environ 1,5% en poids de la composition totale et dans laquelle ledit polymère filmogène comprenant un squelette de polypropylène-glycol (PPG) est un polymère de polyuréthane formé par la réaction d'un polypropylène glycol diol et d'un diisocyanate, présent dans une quantité d'environ 0,1 % à environ 1 % en poids de la composition totale.

3. Composition selon les revendications 1 ou 2, dans laquelle ledit polypropylène glycol diol a la structure : 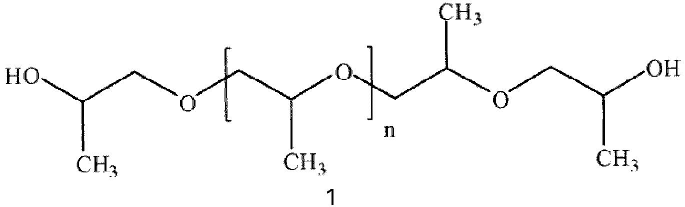 où « n » est un nombre entier de 1 à 100.

4. Composition selon les revendications 1 à 3, dans laquelle « n » est un nombre entier de 5 à 20.

5. Composition selon les revendications 1 à 4, dans laquelle ledit diisocyanate est du méthylène bis(4-cyclohexyl-isocyanate) (SMDI), dans laquelle ladite huile non volatile comprend une huile d'ester et dans laquelle ladite huile non volatile comprend une huile de silicone.

6. Composition selon les revendications 1 à 5, dans laquelle la composition cosmétique à base de poudre longue tenue se présente sous la forme d'une poudre comprimée.

7. Composition selon les revendications 1 à 6, dans laquelle l'huile non volatile comprend un benzoate d'alkyle en C₁₂₋₁₅ ou un mélange d'une huile de silicone et d'un benzoate d'alkyle en C₁₂₋₁₅.

8. Composition cosmétique à base de poudre longue tenue selon les revendications 1 à 7, dans laquelle :
a. une ou plusieurs des poudres cosmétiques comprennent de la séricite et au moins une autre poudre cosmétique choisie dans le groupe constitué du talc, de la poudre de nylon, du lauroyl lysine, de l'oxyde de zinc, du dioxyde de titane, d'oxydes de fer et de combinaisons de ceux-ci ;
b. l'élastomère thermoplastique comprend un copolymère bloc de styrène-isobutylène-styrène (SIBS) ;
c. le polymère filmogène comprend un copolymère PEG-12/SMDI ;
dans laquelle la composition se présente sous la forme d'une poudre qui présente une tenue plus longue sur la peau humaine qu'une composition identique par ailleurs, qui n'inclut pas ledit copolymère bloc SIBS, ladite huile non volatile qui ne s'évapore pas facilement à la température de la pièce et à la pression atmosphérique ni ledit copolymère PEG-12/SMDI.

9. Composition selon la revendication 8, dans laquelle la composition cosmétique à base de poudre longue tenue se présente sous la forme d'une poudre comprimée.

10. Composition selon la revendication 15, dans laquelle l'huile non volatile comprend un benzoate d'alkyle en C₁₂₋₁₅ ou un mélange d'une huile de silicone et d'un benzoate d'alkyle en C₁₂₋₁₅.

11. Procédé pour fournir à la peau un cosmétique à base de poudre longue tenue et résistant au transfert, consistant à appliquer sur la peau une composition comprenant :
a. d'environ 75 % à environ 95 % en poids d'un ou plusieurs cosmétiques à base de poudre ;
b. d'environ 0,01 % à environ 5 % en poids d'un élastomère thermoplastique comprenant un copolymère bloc de styrène-isobutylène-styrène (SIBS) ;
c. d'environ 1 % à environ 20 % en poids d'une huile non volatile ; et
d. d'environ 0,01 % à environ 5 % en poids d'un polymère filmogène comprenant un squelette de polypropylène-glycol (PPG) ;
laquelle composition se présente sous la forme d'une poudre qui présente une tenue plus longue sur la peau humaine qu'une composition identique par ailleurs qui n'inclut pas ledit copolymère bloc SIBS, ladite huile non volatile ni ledit polymère filmogène comprenant un squelette de polypropylène-glycol (PPG).

12. Procédé selon la revendication 11, dans lequel ledit polymère filmogène comprenant un squelette de polypropylène-glycol (PPG) est un polymère de polyuréthane formé par la réaction d'un polypropylène glycol diol et d'un diisocyanate, dans lequel ledit polypropylène glycol diol a la structure : 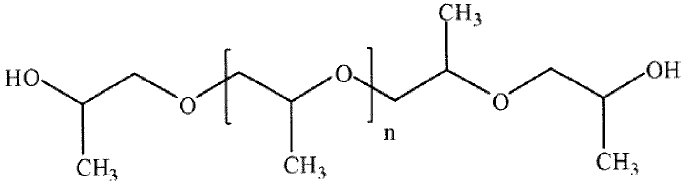 où « n » est un nombre entier de 1 à 100 et dans lequel ledit diisocyanate est du méthylène bis(4-cyclohexyl-isocyanate) (SMDI).

13. Procédé selon les revendications 11 à 12, dans lequel ladite huile non volatile comprend une huile d'ester et dans lequel ladite huile non volatile comprend une huile de silicone.

14. Procédé selon les revendications 11 à 13, dans lequel la composition cosmétique à base de poudre longue tenue se présente sous la forme d'une poudre comprimée.

15. Procédé selon les revendications 11 à 14, dans lequel l'huile non volatile comprend un benzoate d'alkyle en C₁₂₋₁₅ ou un mélange d'une huile de silicone et d'un benzoate d'alkyle en C₁₂₋₁₅.
